# EUROPEAN PATENT SPECIFICATION

(11) **EP 1 450 895 B1**
(45) Date of publication and mention of the grant of the patent: **17.05.2006**
(21) Application number: 02794091.5
(22) Date of filing: 02.12.2002
(51) Int. Cl.: A61N 1/05

(54) **SHAPED LEAD WITH ELECTRODES**
GEFORMTE ZULEITUNG MIT ELEKTRODEN
CONDUCTEUR CONFORME DOTE D'ELECTRODES

(30) Priority: 03.12.2001 US 337224 P; 31.10.2002 US 284710
(43) Date of publication of application: 01.09.2004
(73) Proprietor: MEDTRONIC, INC., Minneapolis, Minnesota 55432-5604 (US)
(72) Inventor: WARMAN, Eduardo, N., Maple Grove, MN 55369 (US); BONNER, Mathew, D., Plymouth, MN 55447 (US); WHITMAN, Teresa, Dayton, MN 55327 (US); BROWN, Mark, L., North Oaks, MN 55127 (US)
(74) Representative: Hughes, Andrea Michelle
(86) International application number: PCT/US2002/038319
(87) International publication number: WO 2003/047687

(56) References cited:
- US-A- 3 903 897
- US-A- 5 713 932
- US-A- 6 021 354
- US-B1- 6 201 994

## Description

### TECHNICAL FIELD

The present invention relates generally to discrimination of cardiac events, and in particular, the present invention relates to cardiac events detected by an atrial electrogram (A-EGM).

### BACKGROUND

Many medical devices, such as implantable pacemakers, pacemaker-cardioverter-defibrillators, other implantable cardioverter-defibrillators, and cardiac monitors, are designed to sense and validly distinguish P-waves from R-waves. Such devices may include one or more electrodes proximate to an atrium of the heart and one or more electrodes proximate to a ventricle of the heart. The signal received via the atrial electrode is the atrial electrogram, or A-EGM, and the signal received via the ventricular electrode is the ventricular electrogram, or V-EGM. The A-EGM and V-EGM reflect the electrical activity of the atrium and the ventricle, respectively.

The A-EGM and V-EGM may be used, for example, to record data about cardiac activity, or to discriminate atrial and ventricular tachyarrhythmias, or to time therapy operations, e.g., atrial and/or ventricular pacing in synchrony with the sensed underlying heart rhythm. Typically, P-waves are sensed in the A-EGM using a unipolar or bipolar atrial lead having one or more pace/sense electrodes in contact with the atrium. Detection of a P-wave may be referred to as an A-SENSE. The R-waves are detected in the V-EGM using a unipolar or bipolar ventricular lead having one or more pace/sense electrodes in contact with the ventricle. Detection of an R-wave may be referred to as a V-SENSE.

In diagnosis and treatment of cardiac conditions, it is important that the A-EGM provide a true representation of the atrial electrical activity. One problem associated with sensing P-waves in the atrium involves the relatively large signal amplitude associated with R-waves generated within, and any pacing pulses delivered to, the ventricle. Because of this large signal magnitude, an electrode positioned in the atrium may sense the R-wave and/or pacing pulses, which are then inappropriately interpreted as a P-wave. In other words, ventricular electrical activity may be reflected in the A-EGM, and this ventricular activity may be wrongly interpreted as atrial activity.

An atrial sensing of a ventricular depolarization is called a far field R-wave. A far field R-wave may lead to "oversensing," because the number of detected atrial events is larger than the total number of actual intrinsic and paced atrial beats. This may, in turn, lead to inappropriate diagnosis of a patient condition. Mistaking a far-field R-wave for a P-wave may result, for example, in an incorrect diagnosis of an atrial arrhythmia. The incorrect diagnosis may in turn cause the device to provide inappropriate treatment for an atrial arrhythmia that did not actually exist.

Oversensing of the far field R-wave using an atrial pacing lead has long been an issue of concern in implantable dual chamber pacing systems as discussed for example, in the background of U.S. Pat. No. 3,903,897. As also disclosed in U.S. Pat. No. 4,825,870, time windows may also be set to detect the near coincidence of the A-SENSE and V-SENSE events and disregard the A-SENSE event. But these approaches may lead to undersensing of legitimate P-waves occurring in a fusion beat with a QRS complex, i.e., mistaking legitimate P-waves for far-field R-waves.

US 6,021,354, US 6,201,994 and US 5,713,932 all disclose atrial pacing leads with means for ensuring the lead is correctly positioned.

One approach to minimizing the amplitude of the far field R-wave in the A-EGM is to minimize the far field QRS spatial gradient propagated to the atrium through the use of closely spaced atrial electrode pairs carefully oriented in the atrium as shown, for example, in U.S. Pat. No. 4,365,639. Both approaches of the '897 and '639 patents are also directed to optimizing the A-EGM sensing performance of the so-called "single pass A-V lead," where the bipolar or multipolar atrial sense electrodes are positioned proximally on a single atrioventricular (A-V) lead body and effectively float in atrial blood in the right atrial chamber. In a further approach, it has long been desired to detect the far field P-wave from a ventricular electrode through suitable filtering and timing as described in the Greenhut, S. E. et al. article, "Detection ofAtrial Activation by Intraventricular Electrogram Morphology Analysis: A Study to Determine the Feasibility of P Wave Synchronous Pacing from a Standard Ventricular Lead," PACE 1993; 16:1293-1303.

Despite the prior art attempts to reduce oversensing, improvement is still needed.

### SUMMARY

In general, the present invention presents an apparatus for reducing far-field R-wave sensing as defined by Claim 1. The invention involves positioning one or more electrodes within the superior vena cava (SVC) in such a way that the electrodes may be separated by a very small distance such as a spacing of less than 2 mm, for example. Studies have shown that positioning the electrodes in this way reduces the far-field R-wave sensing by a factor of three compared to conventional tip/ring configurations positioned within the atrium.

One of the electrodes is positioned in contact with the tissue of the SVC. Another is positioned to be out of contact with the tissue, and "floating" in the blood flowing through the SVC. As a result, each electrode generates a distinct voltage signal as a function of the electrical activity sensed by each electrode, even though the electrodes are in close proximity to one another. When the signal from one electrode is subtracted from the other, the far-field R-wave in the difference signal is substantially reduced.

In one embodiment, the invention is directed to an apparatus comprising a lead body, a first electrode coupled to the lead body, and a second electrode coupled to the lead body. The lead body is shaped to position the first electrode against cardiac tissue, such as tissue in the SVC, and to position the second electrode away from contact with the cardiac tissue. One or both electrodes may protrude from the lead body, and the electrodes may be located close to one another.

The apparatus may further include an anchoring structure proximate to the first electrode. The anchoring structure may promote tissue in-growth adjacent to the first electrode, thereby maintaining contact between the electrode and the tissue. The apparatus may also include a shaping element to shape the lead body to position the first electrode against the cardiac tissue and to position the second electrode away from contact with the cardiac tissue.

Signals from the first and second electrodes may be carried by conductors to an implantable medical device such as a pacemaker-cardioverter-defibrillator or a medical monitor. The implantable medical device may generate a difference signal as a function of the difference between the signals from the first and the second electrodes. The difference signal may have a substantially reduced far-field R-wave, as compared with the signals from the first and the second electrodes. Because of the reduced far-field R-wave in the difference signal, conventional processing techniques are less likely to mistake the far-field R-wave for a P-wave.

The invention may be used in a method comprising introducing a lead that includes a lead body and at least a first electrode and a second electrode proximate to cardiac tissue. The method further includes adjusting the shape of the lead to position the first electrode against the tissue and to position the second electrode away from contact with the tissue. The lead may be introduced proximate to cardiac tissue by insertion into a superior vena cava, for example, or by placing the lead epicardially.

The method may comprise locating a first electrode on a lead body and locating a second electrode on the lead body. The first and second electrodes are so located that when the first electrode is placed in contact with cardiac tissue, the second electrode is not placed in contact with the tissue.

The method of use may also comprise receiving a first signal from a first electrode in contact with cardiac tissue, and receiving a second signal from a second electrode not in contact with the tissue. The second electrode is less than 9 mm from the first electrode. The method also includes generating a third signal as a function of the difference between the first and second signals.

The details of one or more embodiments of the invention are set forth in the accompanying drawings and the description below. Other features, objects, and advantages of the invention will be apparent from the description and drawings, and from the claims.

### BRIEF DESCRIPTION OF DRAWINGS

FIG. 1 is a schematic diagram of an implantable medical device having an electrode configuration according to the present invention.
FIG. 2A is a schematic diagram of an electrode configuration according to the present invention.
FIG. 2B is a schematic diagram of an electrode configuration according to the present invention.
FIG. 3 is a schematic diagram of an implantable medical device having an electrode configuration according to an alternate embodiment of the present invention.
FIG. 4 is an enlarged view of a mesh sleeve positioned along a lead body according to the present invention.
FIG. 5 is a schematic diagram of an implantable medical device having an electrode configuration according to an alternate embodiment of the present invention.
FIG. 6 is a flow diagram illustrating techniques for using the signals generated by the electrodes to reject far-field R-waves, according to the present invention.

### DETAILED DESCRIPTION

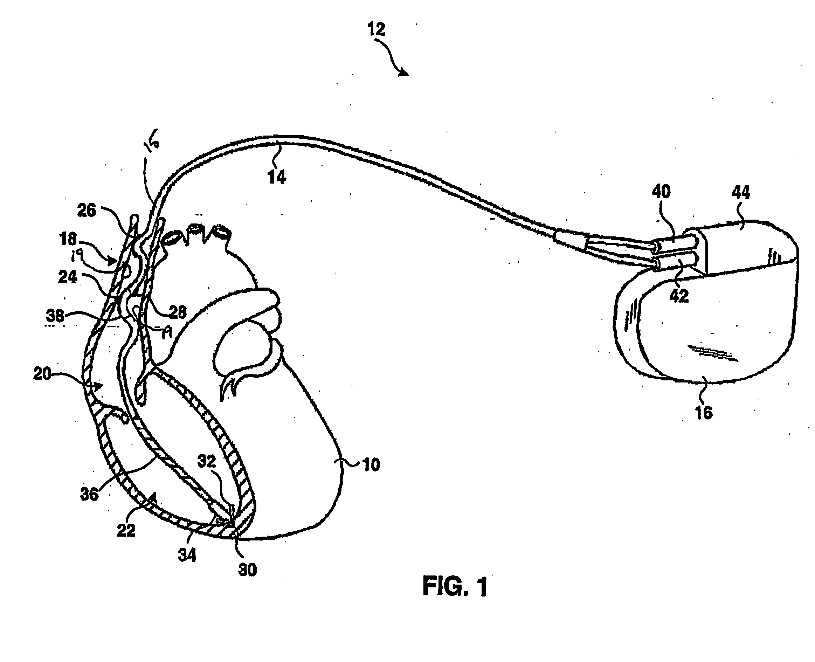

FIG. 1 is a schematic diagram of an implantable medical device having an electrode configuration according to the present invention. As illustrated in FIG. 1, an implantable medical device (IMD) 12 includes a lead 14 coupled to and extending from a hermetically sealed housing 16 and extending within a heart 10. IMD 12 will be described as a dual-chamber pacemaker-cardioverter-defibrillator, although it is understood that the invention is not intended to be limited to application with a pacemaker-cardioverter-defibrillator. IMD 12 may be, for example, a pacemaker that provides pacing but no cardioversion or defibrillation therapies, or a medical monitor that provides no pacing, cardioversion or defibrillation therapies.

As illustrated in FIG. 1, lead 14 extends through superior vena cava (SVC) 18 of heart 10, though a right atrium 20 and into a right ventricle 22 of heart 10. A lead similar to lead 14 is described in U.S. Patent No. 6,201,994.

Lead 14 includes one or more mechanisms for maintaining an atrial pacing/sensing electrode 24 adjacent stimulable tissue within SVC 18. For example, one or more shaped portions 26 of lead 14 may be shaped or curved to provide a means of retention of lead 14 within SVC 18. Shaped portion 26 may, for example, cause lead 14 to have an S-shaped curve within SVC 18. Any of several shaping elements, described in more detail below, may be employed to cause the shape of lead 14 to push against wall 19 of SVC 18, thereby holding lead 14 in place by compression and friction. Curves may extend laterally, and have a width of at least 2 cm, and preferably more to maintain the position of lead 14 within SVC 18. Examples of other position retention techniques will be described below.

As illustrated in FIG. 1, lead 14 also includes electrodes 24, 28, spaced along a lead body 15 of lead 14 so that at least one electrode 24 is positioned against tissue along wall 19 for pacing and for sensing electrical activity, and a second electrode 28 is suspended within the blood within wall 19 for sensing. As shown in FIG. 1, electrodes 24, 28 may be offset from one another, with electrode 24 projecting from lead body 14 more distally along lead 14 from housing 16 than electrode 28. In one embodiment, second electrode 28 is on a different side of lead 14 from first electrode 24, as shown in FIG. 1. The two electrodes 24, 28 may be placed in an orthogonal relationship using the S-shape of lead 14 to ensure that one electrode 24 is contacting active tissue and the other electrode 28 is floating within bodily fluids. Additional electrodes (not shown in FIG. 1) may also be provided on lead 14 to be positioned proximate to SVC or atrium 20.

In one embodiment of the invention, electrodes 24 and 28 may be unipolar electrodes. Electrodes 24 and 28 sense electrical activity of heart 10 and generate voltage signals as a function of the sensed electrical activity. The voltage signals generated by each electrode may be with respect to housing 16, which serves as a common electrode.

Electrodes 24, 28 provided within the shaped portion of lead 14 may be of any type of electrode known in the art for pacing and sensing within the atrium, including any of the ring electrodes. Also provided adjacent the electrodes may be monolithic controlled release devices (MCRDs) to release steroids or other types of biologically active agents into the body.

In one embodiment, electrodes 24, 28 are of the type shown in commonly assigned U.S. Patent No. 5,772,693 to Brownlee. Electrodes 24 and 28, which form a projection or prominence extending outward from lead body 15, enhance detection of atrial depolarization and the pacing function in atrial chamber 20. In order to increase the probability of atrial wall contact, electrode 24 may extend outward from lead body 15 as shown in FIG. 1. In the same way, electrode 28 may also protrude outward from lead body 15 to ensure that electrode 28 is suspended in body fluids.

In general, it is usually more desirable and more efficient to position electrodes 24, 28 in SVC 18 than in right atrium 20, since atrium 20 may have an irregular shape, and the shape of atrium 20 changes as atrium 20 contracts and fills during a cardiac cycle. In addition, atrium 20 may include more connective tissue than SVC 18, and as a result, there is an enhanced risk that both electrodes 24 and 28 may come in contact with tissue in atrium 20.

SVC 18, by contrast, generally has a more regular, cylindrical shape. SVC 18, like atrium 20, presents a good site for sensing atrial activations and for delivering atrial paces. As a practical matter, it may be easier to shape lead 14 to retain a position in SVC 18 than in atrium 20. The walls of SVC 18 are somewhat flexible, and may hold shaped lead 14 in place by compression and friction. In addition, it may be easier to shape lead 14 to position electrode 24 in contact with the tissue of SVC 18, while positioning electrode 28 away from contact with the tissue of SVC 18, because less connective tissue is present in SVC 18.

In the embodiment of the invention shown in FIG. 1, lead 14 is a single-pass defibrillation lead. Lead 14 includes a tip electrode 30 which may include tines 32 or another fixation mechanism. Lead 14 may also include a ring electrode 34 and a defibrillation coil 36 positioned within right ventricle 22. The distance from the shaped portion of lead 14 to the lead tip may be approximately 18-20 cm.

In another embodiment of the invention, lead 14 includes an anchoring structure 38 for promoting tissue in-growth adjacent to first electrode 24. This anchoring structure may include the use of a Dacron mesh sleeve. In another embodiment, the anchoring structure may include a layer or coating formed of expanded polytetrafluoroethylene (ePTFE) having a pore size sufficient to promote tissue in-growth as described in commonly-assigned US patent application publication number 20020147486 filed April 10, 2001. Examples of anchoring structures for promoting tissue in-growth will be described in more detail below. Any other structure of promoting tissue in-growth in proximity to electrode 24, or otherwise anchoring electrode 24 in position, may be used in the alternative.

Anchoring structures keep electrode 24 in contact with cardiac tissue, while at the same time maintaining lead 14 in a position so that electrode 28 remains floating in fluid, and not in contact with cardiac tissue. As will be described below, maintaining one electrode in contact with tissue and the other electrode not in contact with tissue is useful in rejecting far-field R-waves. Anchoring structures and shaping elements help assure that the placement of lead 14 and electrodes 24, 28 will not be disturbed.

Lead 14 is coupled to housing 16 via connectors 40 and 42, which in turn are coupled to connector block 44. Inside lead body 15 of lead 14, separate insulated elongated conductors may be coupled to electrodes 24, 28 and may extend proximally inside lead body 15 to IMD 12. Because electrodes 24, 28 are coupled to housing 16 via independent conductors, IMD 12 may distinguish separate senses from electrodes 24 and 28. In addition, a pacing stimulus may be supplied to electrode 24 without supplying the stimulus to electrode 28.

Housing 16 may include one or more sense amplifiers that detect atrial and ventricular activations sensed via electrodes 24, 28, 30, 34. Housing 16 may also include a processor to detect the atrial and ventricular activations and sense arrhythmias. The processor may also control delivery of appropriate pacing or defibrillation therapies to heart 10 via lead 14 in response to sensed arrhythmias.

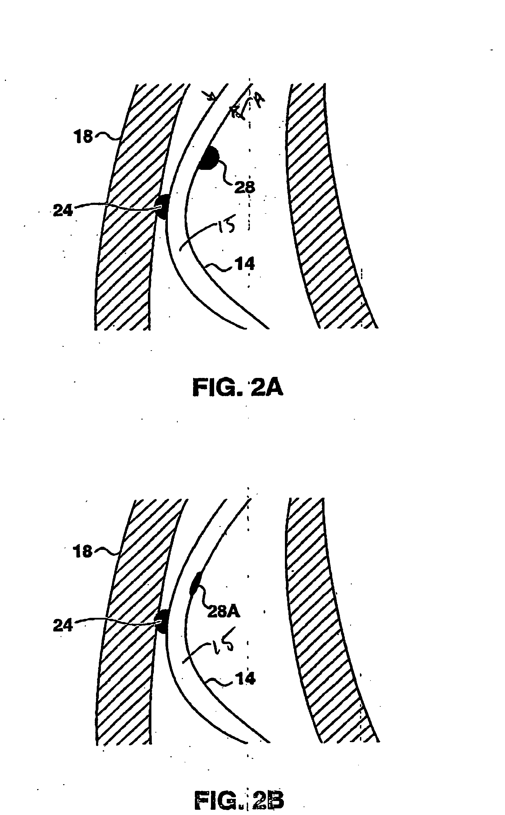

FIG. 2A is a schematic diagram of an electrode configuration according to the present invention. As illustrated in FIG. 2A, electrode 24 is positioned more distally along lead body 15 from housing 16 than electrode 28 and in contact with the tissue of SVC 18. Because of the curvature of lead 14, and because electrode 28 is on the side of lead 14 opposite that of electrode 24, electrode 28 is not in contact with the tissue of SVC 18. In other words, electrodes 24 and 28 are configured on opposite sides of lead body 14 such that, when electrode 24 is in contact with the tissue, electrode 28 cannot be in contact with the tissue. In addition, in the embodiment illustrated in FIG. 2A, both electrode 24 and electrode 28 extend outward from lead body 15.

The distance between electrodes 24 and 28 may be less than 9 mm, and in general, it may be advantageous to have electrodes 24 and 28 very close to one another. In one embodiment, the electrodes are approximately 1.7 to 2 mm apart. In another embodiment described below, the electrodes are separated by the thickness of lead body 15, shown by arrows A.

FIG. 2B is a schematic diagram of an electrode configuration according to the present invention. Similar to FIG. 2A, electrodes 24 and 28A of FIG. 2B are positioned on opposite sides of lead body 14, with electrode 24 located more distally from housing 16 along lead body 15 than electrode 28A. Also similar to FIG. 2A, electrode 24 projects from lead body 15 and is in contact with the tissue of SVC 18. Unlike FIG. 2A, however, electrode 28A is recessed within lead body 15. Because electrode 24 extends outward from lead body 15, there is an improved chance that electrode 24 will come in contact with the tissue along wall 19 of SVC 18. Because electrode 28A is recessed within lead body 15, there is a reduced risk that electrode 28A will come in contact with the tissue. It is understood that although electrode 24 is shown in FIG. 2A and FIG. 2B as extending outward from lead body 15 to increase the likelihood of contact of electrode 24 with SVC 18, the present invention is not intended to be limited to having electrode 24 extend outward from lead body 15. Rather, according to the present invention electrode 24 may be recessed within lead body 15 or positioned along the same plane as lead body 15 if desired.

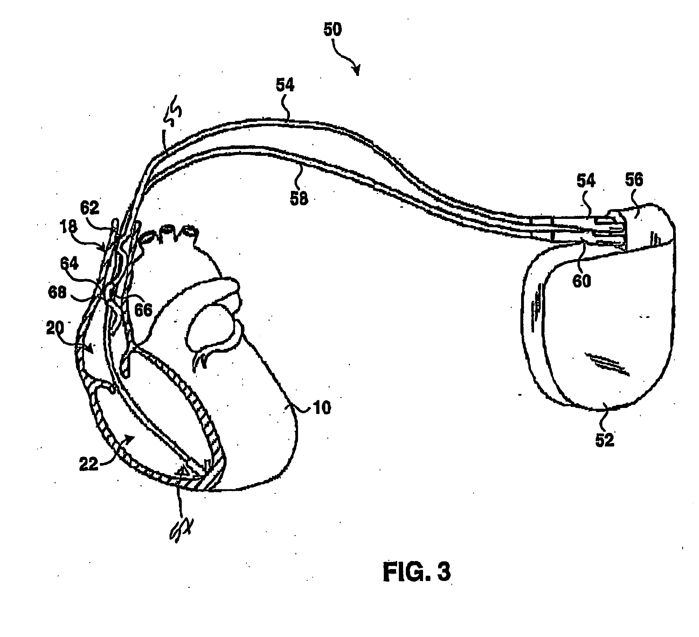

FIG. 3 is a schematic diagram of an implantable medical device having an electrode configuration according to an alternate embodiment of the present invention. As illustrated in FIG. 3, according to the present invention, an IMD 50, such as a dual-chamber pacemaker with no defibrillation capability, includes an atrial lead 54 coupled to a hermetically sealed housing 52 via connectors 54 and 60 coupled to a connector block 56. Atrial lead 54 extends from connector block 56 through SVC 18. As shown in FIG. 3, atrial lead 54 may also extend into right atrium 20. Unlike lead 14 shown in FIG. 1, however, atrial lead 54 does not extend into right ventricle 22. Rather, ventricular lead 58, coupled to housing 52 via connector 60 and connector block 56, extends through SVC 18 and right atrium 20 and into right ventricle 22. Ventricular lead 58 may be a conventional ventricular lead, with bipolar or unipolar electrodes on a distal end 57 of lead 58.

Atrial lead 54, like lead 14, includes a lead body 55 and may include one or more shaping elements, such as shaped portion 62 for maintaining an atrial pacing electrode 64 adjacent stimulable tissue within SVC 18. Atrial lead 54 may also include a second electrode 66, which is suspended within the blood. The shape of atrial lead 54 ensures that electrode 64 is contacting active tissue and the electrode 66 is floating within bodily fluids. Additional electrodes (not shown in FIG. 3) may also be provided. Electrodes 64, 66 may be of any type of electrode known in the art for pacing and sensing within the atrium,

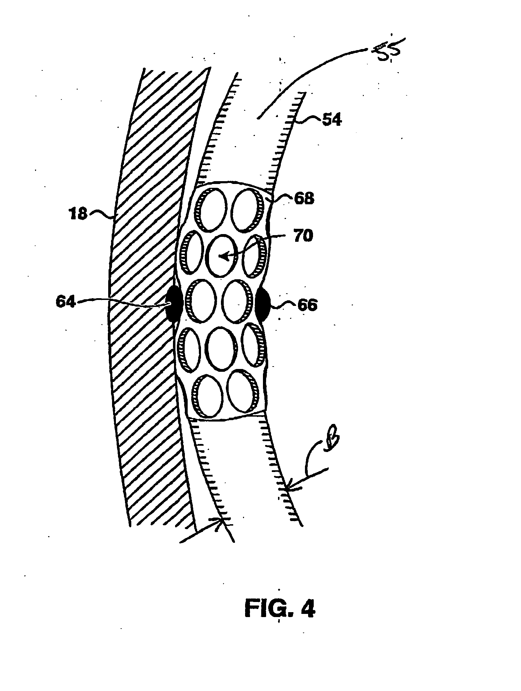

Electrodes 64 and 66 are depicted as protruding from directly opposite sides of lead 54. This arrangement will be shown in more detail in FIG. 4. Electrodes 24 and 28 in FIGS. 1 and 2A, by contrast, protrude from opposite sides of lead 14 but are offset from one another. The different arrangements allow for different spacings between the electrode that is in contact with the tissue and the electrode that is suspended in the blood. The invention encompasses embodiments with a variety of electrode spacings.

Lead 54 may include a structure 68 for promoting tissue in-growth adjacent to electrode 64. An example of such a structure, which may be similar to structure 38 described above in connection with FIG. 1, will be shown in more detail in FIG. 4.

The S-shaped configuration of leads 14 and 54 is exemplary. Other shapes, such as coils or zigzags may be used to retain the lead within SVC 18. A lead may include any number of shaped portions, for example. An S-shaped lead may include a single undulation, or more than one undulation, like leads 14 and 54 shown in FIGS. 1 and 3.

Following insertion of the lead into SVC 18, the shape of the lead may be adjusted using any of several techniques. For example, the shaped portion of a lead may be generated using a sleeve made of a heat-deformable material such as polyurethane. In one embodiment, the sleeve may be adapted to be slid over and around the lead body, and affixed thereto using an adhesive or another means of fixation. This may be provided over the lead body, which may be made of another biocompatible material such as silicone or a biocompatible polymer. The sleeve may be flexible enough to permit smooth introduction of the lead into heart 10, but may assume a curved configuration following introduction. More than one such sleeve may be affixed to a lead.

In another embodiment of the present invention, the lead may include a shape memory element, such as a wire made of nitinol. The shape memory element may be elongated for introduction, and may assume a pre-defined shape following introduction. The invention is not limited to any particular mechanism for adjusting the shape of the lead into a retaining shape.

FIG. 4 is an enlarged view of a mesh sleeve positioned along a lead body according to the present invention. As illustrated in FIG. 4, a mesh sleeve 68 may be used to promote tissue in-growth. FIG. 4 further illustrates first electrode 64 positioned in contact with tissue of SVC 18, and a second electrode 66 positioned on the opposite side of lead body 55, suspended in bodily fluids. Because electrodes 64 and 66 are on opposite sides of lead body 55, when electrode 64 is in contact with the tissue of SVC 18, electrode 66 cannot be in contact with the tissue. Electrodes 64 and 66 protrude from mesh sleeve 68.

In the embodiment shown in FIG. 4, electrodes 64, 66 are separated by the thickness of lead body 55, shown by arrows B. As noted above, however, the distance between the electrodes may be less than 9 mm, and in general, it may be advantageous to have the electrodes very close to one another.

As noted above, sleeve 68 may be formed of a Dacron mesh sleeve, or may include a layer or coating formed of ePTFE. Sleeve 68 may be affixed to lead 54 using an adhesive or another means of fixation. The dimensions of sleeve 68, and the shape and size of openings 70 of the mesh, are illustrative. Any dimensions, size or shape may be employed to promote tissue in-growth adjacent to electrode 64. When tissue grows into mesh sleeve 68, lead 54 acquires enhanced anchoring, and electrode 64 is less likely to be disturbed from contact with the tissue. Similarly, tissue in-growth also prevents electrode 66 from coming in contact with the tissue.

The invention is not limited to the leads shown in FIGS. 1-4. Rather, the invention may employ any lead configured to position one atrial electrode in contact with the cardiac tissue and another electrode floating in blood. As noted above, the lead may employ any of a variety of shapes to bring at least one electrode in contact with the tissue and at least one electrode not in contact. The lead may include any configuration or mechanism to maintain the position of lead, to keep one electrode in contact and at least one electrode floating. The lead may also include an anchoring mechanism such as the sleeve described above, which enhances position retention with tissue in-growth. The lead may include any combination of shaping and anchoring mechanisms.

It has been discovered that electrodes positioned as shown in FIGS. 1-4 generate signals that may be used to reject far-field R-waves. In particular, electrodes placed in SVC 18 or right atrium 20 close to one another, with one electrode in contact with the tissue and the other not in contact with the tissue, generate signals that may be used to reject far-field R-waves.

In general, cardiac electrical activity may have an atrial origin or a ventricular origin. When an electrode in the SVC detects a cardiac electrical activity, the electrode generate voltage signals as a function of the sensed electrical activity. The ventricular electrical activity is generally of greater amplitude than the atrial electrical activity, but the ventricular activity originates a greater distance away from the electrodes in the SVC than the atrial activity. Whether an electrode is in contact with tissue or not also affects the amplitude of the signal generated by the electrode, because the conductivity of tissue is different from the conductivity of blood.

Because of these factors, the electrode in contact with the tissue generates a stronger signal with larger amplitude than the electrode that is not touching the tissue. In particular, the electrode in contact with the tissue may generate a signal with a prominent P-wave and a pronounced far-field R-wave. By contrast, the electrode not in contact with the tissue may generate a signal with a small P-wave but with a fairly pronounced far-field R-wave.

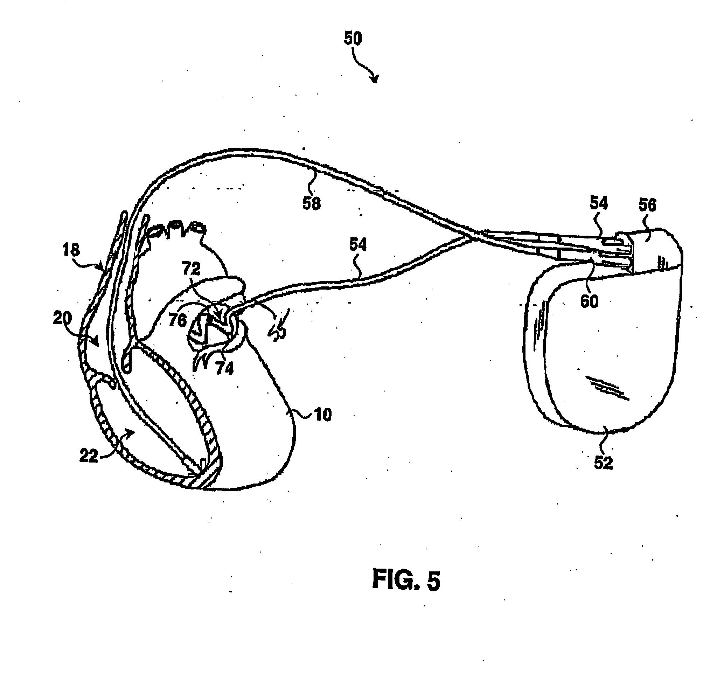

FIG. 5 is a schematic diagram of an implantable medical device having an electrode configuration according to an alternate embodiment of the present invention. The implanatable medical device shown in FIG. 5 is similar to the device shown in FIG. 3. However, unlike FIG. 3, atrial lead 54 is deployed epicardially, rather than endocardially. In particular, atrial lead 54 is deployed on the surface of the left atrium 72.

Atrial lead 54 includes one or more shaping elements (not shown in FIG. 5) that cause lead body 55 to have a zigzag shape. The zigzag shape causes lead 54 to lie flat against left atrium 72 between the cardiac tissue and a lining of the pericardial sac (not shown). On the side of lead 54 facing the tissue is an electrode (not shown) contacting active tissue. On the opposite side of lead 54 is an electrode 76 that is not in contact with the tissue. The electrode in contact with the tissue may be directly opposite electrode 76, or the electrodes may be offset from one another.

Atrial lead 54 may be placed in contact with left atrium 72 via any surgical technique, including "keyhole" or other minimally invasive surgery. The surgeon may incise the pericardial sac for introduction of lead 54 against cardiac tissue. The shape of lead 54, particularly the proximal end of lead 54, may be adjusted using any of several techniques. The shaped end of lead 54 tends to keep the lead from turning, thereby keeping one electrode in contact with cardiac tissue and the other electrode away from contact.

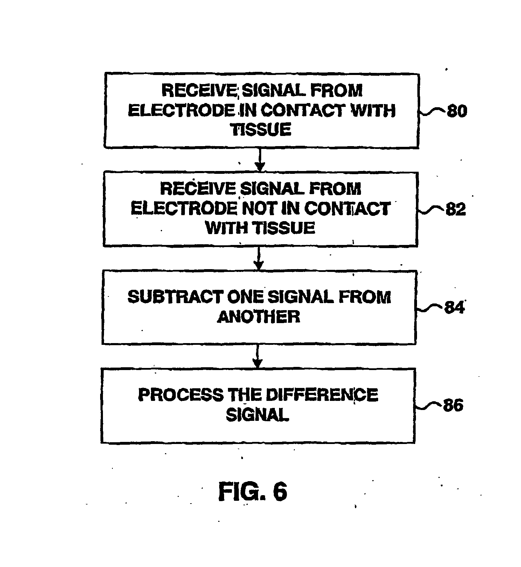

FIG. 6 is a flow diagram illustrating techniques for using the signals generated by the electrodes to reject far-field R-waves, according to the present invention. Circuitry in the housing of the IMD receives signals from the electrode in contact with the tissue and the electrode not in contact with the tissue (80, 82) and subtracts one processing of the signals received from the other (84). The subtraction may be performed using analog or digital circuitry. For example, the signals may be received by a differential amplifier that generates a difference signal. The subtraction may be accompanied by a time offset of one of the signals, so that the far-field R-waves in the signals are temporally aligned.

When the signal from the non-contacting electrode is subtracted from the signal from the contacting electrode, the result is a difference signal with a prominent P-wave but a substantially reduced far-field R-wave. This difference signal, which has a substantially reduced far-field R-wave, may be processed with conventional techniques. Because of the reduced far-field R-wave in the difference signal, the far-field R-wave is less likely to be mistaken for a P-wave. In some animal studies, these techniques have reduced far-field R-waves by a factor of three.

In this way, the present invention can be used for discriminating cardiac events by receiving a first signal from a first electrode in contact with cardiac tissue, receiving a second signal from a second electrode not in contact with the tissue, with the second electrode being less than approximately 9 mm from the first electrode, for example, and generating a third signal as a function of the difference between the first and second signals.

The invention may offer one or more advantages. The lead and electrode placements described above may reduce the risk of oversensing and undersensing due to far-field R-waves. The techniques for shaping and anchoring the lead help assure that the lead and electrode placements will not be disturbed by the motion of the tissue, the movement of the blood, or the physical activity of the patient.

Various embodiments of the invention have been described. The preceding specific embodiments are illustrative of the practice of the invention. Various modifications may be made without departing from the scope of the claims. For example, the invention may be embodied in an IMD that includes more leads than depicted in the figures, or that includes no ventricular lead of any kind. The invention may also be practiced with an IMD that may deliver a defibrillation shock to the atrium, in which case the lead extending into the SVC may include a defibrillation electrode in addition to pacing and sensing electrodes.

In addition, the invention is not limited to any particular configuration of lead body and electrodes. The lead shapes described above are not the only shapes that may be applied with the invention. In addition, the invention may be practiced with electrodes different from those described above or presented in the figures. These and other embodiments are within the scope of the following claims.

## Claims

1. An implantable medical device (12) comprising
a housing (16);
a lead (14) having a lead body (15), the lead coupled to and extending distally from the housing; said device further comprising
an electrode configuration for increased discrimination of cardiac events, wherein a first electrode and a second electrode (24, 28) are coupled to said lead body of the implantable device and the lead body is shaped to position the first electrode (24) against cardiac tissue and to position the second electrode (28) away from contact with the cardiac tissue, **characterized in that** the first and second electrode are positioned along opposite sides of the lead body, and wherein the first electrode extends outward from the lead body.

2. The implantable medical device of claim 1, wherein the cardiac tissue comprises tissue of the superior vena cava.

3. The implantable medical device of claim 1, wherein the second electrode (28) extends outward from the lead body.

4. The implantable medical device of claim 1, wherein a distance between the first electrode and the second electrode is less than 9 mm.

5. The implantable medical device of claim 1, wherein a distance between the first electrode and the second electrode is less than 2 mm.

6. The implantable medical device of any preceding claim, further comprising an anchoring structure (38) proximate to the first electrode to promote tissue in-growth adjacent to the first electrode.

7. The implantable medical device of claim 6, wherein the anchoring structure comprises a Dacron mesh.

8. The implantable medical device of claim 6, wherein the anchoring structure comprises a sleeve positioned about the lead body.

9. The implantable medical device of claim 6, wherein the anchoring structure includes a layer of expanded polytetrafluoroethylene.

10. The implantable medical device of claim 1, further comprising a shaping element (62) to shape the lead body to position the first electrode against the cardiac tissue, and to position the second electrode away from contact with the cardiac tissue.

11. The implantable medical device of claim 10, wherein the shaping element comprises a sleeve made of heat-deformable material.

12. The implantable medical device of claim 11, wherein the heat-deformable material is polyurethane.

13. The implantable medical device of claim 11, wherein the shaping element includes a shape memory element.

14. The implantable medical device of claim 13, wherein the shape memory element is nitinol.

15. The implantable medical device of claim 1, wherein the lead body includes a distal end, the implantable medical device further comprising a third electrode (32) proximal to the distal end.

16. The implantable medical device of claim 1, further comprising a defibrillation coil coupled to the lead body.

17. The implantable medical device of any preceding claim, wherein the device is adapted to generate a difference signal as a function of a difference between a first signal corresponding to the first electrode and a second signal corresponding to the second electrode.

## Patentansprüche

1. Implantierbare medizinische Vorrichtung (12) mit
einem Gehäuse (16);
einer Leitung bzw. Zuleitung (14) mit einem Leitungskörper (15), wobei die Leitung mit dem Gehäuse gekoppelt ist und sich distal von diesem erstreckt; wobei die Vorrichtung ferner
eine Elektrodenkonfiguration zur verbesserten Diskriminierung bzw. Unterscheidung von Herzereignissen aufweist, wobei eine erste Elektrode und eine zweite Elektrode (24, 28) gekoppelt sind mit dem Leitungskörper der implantierbaren Vorrichtung, und der Leitungskörper geformt ist zur Positionierung der ersten Elektrode (24) gegen bzw. an Herzgewebe, und zur Positionierung der zweiten Elektrode (28) ausser Kontakt mit dem Herzgewebe,
**dadurch gekennzeichnet, dass**
die erste und die zweite Elektrode entlang entgegengesetzter Seiten des Leitungskörpers positioniert sind, und wobei die erste Elektrode sich von dem Leitungskörper nach aussen erstreckt.

2. Implantierbare medizinische Vorrichtung nach Anspruch 1, bei der das Herzgewebe Gewebe der Vena cava superior umfasst.

3. Implantierbare medizinische Vorrichtung nach Anspruch 1, bei der die zweite Elektrode (28) sich von dem Leitungskörper nach aussen hin erstreckt.

4. Implantierbare medizinische Vorrichtung nach Anspruch 1, bei der ein Abstand zwischen der ersten Elektrode und der zweiten Elektrode weniger als 9 mm beträgt.

5. Implantierbare medizinische Vorrichtung nach Anspruch 1, bei der ein Abstand zwischen der ersten Elektrode und der zweiten Elektrode weniger als 2 mm beträgt.

6. Implantierbare medizinische Vorrichtung nach einem der vorstehenden Ansprüche, ferner mit einer Verankerungsstruktur (38) in der Nähe der ersten Elektrode zur Förderung eines Gewebeeinwachsens in der Nähe der ersten Elektrode.

7. Implantierbare medizinische Vorrichtung nach Anspruch 6, bei der die Verankerungsstruktur ein Dacron-Gitter aufweist.

8. Implantierbare medizinische Vorrichtung nach Anspruch 6, bei der die Verankerungsstruktur eine Hülse bzw. Manschette aufweist, die um den Leitungskörper herum positioniert ist.

9. Implantierbare medizinische Vorrichtung nach Anspruch 6, bei der die Verankerungsstruktur eine Schicht eines expandierten Polytetrafluoroethylens aufweist.

10. Implantierbare medizinische Vorrichtung nach Anspruch 1, ferner mit einem Formungselement (62) zum Formen des Leitungskörpers zur Positionierung der ersten Elektrode gegen das Herzgewebe, und zur Positionierung der zweiten Elektrode ausser Kontakt mit dem Herzgewebe.

11. Implantierbare medizinische Vorrichtung nach Anspruch 10, bei der das Formungselement eine Manschette aus einem wärmeverformbaren Material aufweist.

12. Implantierbare medizinische Vorrichtung nach Anspruch 11, bei der das wärmeverformbare Material Polyurethan ist.

13. Implantierbare medizinische Vorrichtung nach Anspruch 11, bei der das Formungselement ein Formgedächtniselement aufweist.

14. Implantierbare medizinische Vorrichtung nach Anspruch 13, bei der das Formgedächtniselement Nitinol ist.

15. Implantierbare medizinische Vorrichtung nach Anspruch 1, bei der der Leitungskörper ein distales Ende aufweist, und die implantierbare medizinische Vorrichtung ferner eine dritte Elektrode (32) proximal zu dem distalen Ende aufweist.

16. Implantierbare medizinische Vorrichtung nach Anspruch 1, ferner mit einer Defibrillationsspule, die mit dem Leitungskörper gekoppelt ist.

17. Implantierbare medizinische Vorrichtung nach einem der vorstehenden Ansprüche, bei der die Vorrichtung eingerichtet ist zur Erzeugung eines Differenzsignals als Funktion einer Differenz zwischen einem ersten Signal, das zu der ersten Elektrode korrespondiert, und einem zweiten Signal, dass zu der zweiten Elektrode korrespondiert.

## Revendications

1. Dispositif médical implantable (12) comportant :
un boîtier (16),
un raccord (14) ayant un corps de raccord (15), le raccord étant couplé au boîtier et s'étendant de manière distale à partir de celui-ci, ledit dispositif comportant de plus :
une configuration d'électrodes pour une discrimination accrue d'événements cardiaques, dans lequel une première électrode et une deuxième électrode (24, 28) sont couplées audit corps de raccord du dispositif implantable et le corps de raccord est mis en forme pour positionner la première électrode (24) contre le tissu cardiaque et pour positionner la deuxième électrode (28) loin d'un contact avec le tissu cardiaque, **caractérisé en ce que** les première et deuxième électrodes sont positionnées le long des côtés opposés du corps de raccord, et dans lequel la première électrode s'étend vers l'extérieur du corps de raccord.

2. Dispositif médical implantable selon la revendication 1, dans lequel le tissu cardiaque comporte le tissu de la veine cave supérieure.

3. Dispositif médical implantable selon la revendication 1, dans lequel la deuxième électrode (28) s'étend vers l'extérieur du corps de raccord.

4. Dispositif médical implantable selon la revendication 1, dans lequel une distance entre la première électrode et la deuxième électrode est inférieure à 9 mm.

5. Dispositif médical implantable selon la revendication 1, dans lequel une distance entre la première électrode et la deuxième électrode est inférieure à 2 mm.

6. Dispositif médical implantable selon l'une quelconque des revendications précédentes, comportant de plus une structure d'ancrage (38) à proximité de la première électrode afin de favoriser une incarnation de tissu près de la première électrode.

7. Dispositif médical implantable selon la revendication 6, dans lequel la structure d'ancrage se constitue d'une grille de Dacron.

8. Dispositif médical implantable selon la revendication 6, dans lequel la structure d'ancrage comporte un manchon positionné autour du corps de raccord.

9. Dispositif médical implantable selon la revendication 6, dans lequel la structure d'ancrage inclut une couche de polytétrafluoroéthylène expansé.

10. Dispositif médical implantable selon la revendication 1, comportant de plus un élément de mise en forme (62) pour mettre en forme le corps de raccord afin de positionner la première électrode contre le tissu cardiaque, et positionner la deuxième électrode loin d'un contact avec le tissu cardiaque.

11. Dispositif médical implantable selon la revendication 10, dans lequel l'élément de mise en forme comporte un manchon constitué d'un matériau thermiquement déformable.

12. Dispositif médical implantable selon la revendication 11, dans lequel le matériau thermiquement déformable est le polyuréthane.

13. Dispositif médical implantable selon la revendication 11, dans lequel l'élément de mise en forme inclut un élément de mémoire de forme.

14. Dispositif médical implantable selon la revendication 13, dans lequel l'élément de mémoire de forme est le nitinol.

15. Dispositif médical implantable selon la revendication 1, dans lequel le corps de raccord inclut une extrémité distale, le dispositif médical implantable comportant de plus une troisième électrode (32) proximale à l'extrémité distale.

16. Dispositif médical implantable selon la revendication 1, comportant de plus une spirale de défibrillation couplée au corps de raccord.

17. Dispositif médical implantable selon l'une quelconque des revendications précédentes, dans lequel le dispositif est adapté pour générer un signal de différence qui est fonction d'une différence entre un premier signal correspondant à la première électrode et un second signal correspondant à la deuxième électrode.
